# EUROPEAN PATENT SPECIFICATION

(11) **EP 3 839 036 B1**
(45) Date of publication and mention of the grant of the patent: **19.04.2023**
(21) Application number: 19217964.6
(22) Date of filing: 19.12.2019
(51) Int. Cl.: C12M 1/36, G05B 13/04

(54) **A DIGITAL TWIN FOR MONITORING AND CONTROLLING AN INDUSTRIAL BIOPROCESS**
DIGITALER ZWILLING ZUR ÜBERWACHUNG UND STEUERUNG EINES INDUSTRIELLEN BIOPROZESSES
DOUBLE NUMÉRIQUE PERMETTANT DE SURVEILLER ET DE COMMANDER UN BIOPROCÉDÉ INDUSTRIEL

(43) Date of publication of application: 23.06.2021
(73) Proprietor: DSM IP Assets B.V., 6411 TE Heerlen (NL)
(72) Inventor: WU, Liang, 6100 AA Echt (NL); NOORMAN, Hendrik Jan, 6100 AA Echt (NL); HARINGA, Cornelis, 6100 AA Echt (NL)
(74) Representative: DSM Intellectual Property

(56) References cited:
- WO-A1-2018/229802
- Pragyansri Pathi: "Florida State University Libraries Mathematical Modeling of Transport and Reaction in Cellular and Tissue Engineering", , 1 January 2005 (2005-01-01), XP055695228, Retrieved from the Internet: URL:http://diginole.lib.fsu.edu/islandora/ object/fsu:180374/datastream/PDF/view
- LAAKKONEN ET AL: "Modelling local bubble size distributions in agitated vessels", CHEMICAL ENGINEERING SCIENCE, OXFORD, GB, vol. 62, no. 3, 15 December 2006 (2006-12-15), pages 721-740, XP005805322, ISSN: 0009-2509, DOI: 10.1016/J.CES.2006.10.006

## Description

### Field of the invention

The invention relates to a digital twin for monitoring and/or controlling performance of an industrial bioprocess. It further relates to accurately modelling an industrial scale bioprocess and using the model to control the performance of the bioprocess.

### Background art

Bioprocesses are those in which living organisms play a key role in the production of substances. An example of a bioprocess is fermentation, which is extensively used in industrial sectors for production of food, beverages, pharmaceuticals, and the like. The process typically uses one or more microorganism like bacteria, fungi etc. to convert organic substances like sugars, proteins, polysaccharides etc., otherwise called substrates, or the active components in a feed composition. The products of the conversion process range from acids, gases to alcohol, metabolites, enzymes or biomass. The microorganism may be so chosen to provide a desired substance or compound, and the feed composition or the substrate may be specific to the microorganism used in the process. A microorganism may convert at least a portion of the substrate into one or more products.

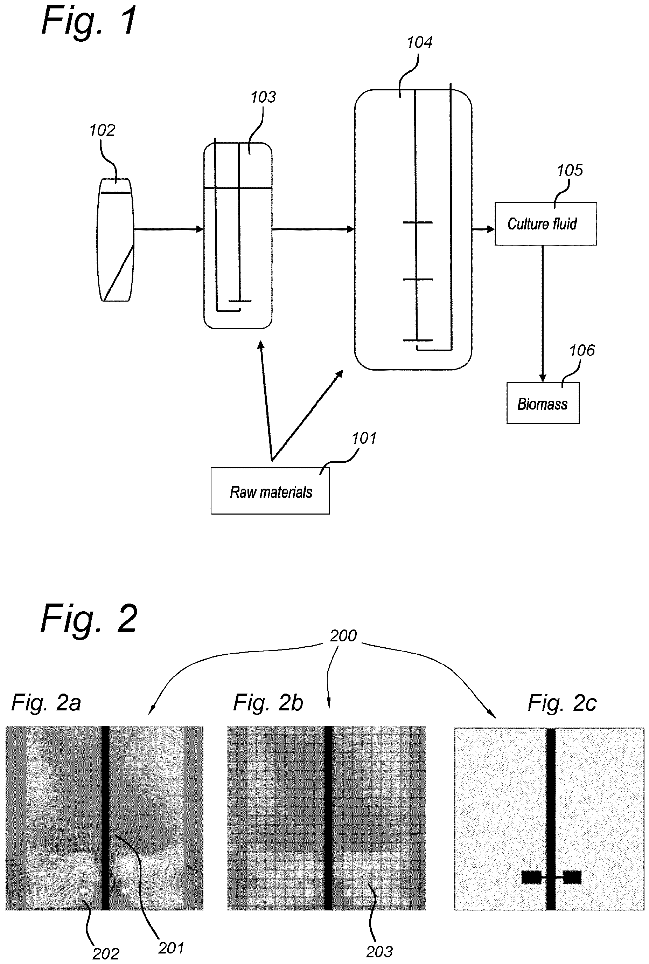

As exemplified in figure 1, an industrial bioprocess can be depicted as a sequence of operations run at industrial scale, where a feed composition (stock culture) is converted and purified into a final form that meets the specifications of the user.

Bioprocesses like fermentation are carried out in bioreactors or fermenters. The development of the inoculum marks the start of fermentation. The feed composition is inoculated with the microorganism. This is implemented by mixing the raw materials (101) with the stock culture (102) in a seed fermenter (103), the output of which is fed to a production fermenter (104). The raw materials are additionally fed to the production fermenter to ensure supply. Microorganisms or biomass (106) is the intended product of fermentation. Examples include single cell protein, yeast, lactobacillus, E. coli, and others. The biomass is obtained by performing cell separation on the culture fluid (105) which is output from the production fermenter (104).
Extensive care is given to process conditions to ensure production of substances/biomass under optimal conditions, for example, their production in desired concentrations. There are a number of parameters which play a role in a fermentation process. These include, but are not limited to, pH, temperature, oxygen supply, glucose content, the initial feed composition or profile, fermentation time, and pressure.

Once the reaction(s) commence, the process may entail a decrease in concentration of the substrate owing to its usage by the microorganism. The latter, on the other hand, may see a growth due to consumption of the substrate, leading to an increase in the end products of the process. This, however, is not a uniform process - for example, the reactions may follow different rates of occurrence, even if performed under similar experimental conditions. In an industrial setting, the regimes for fluid flow and reaction are likely not the same as in a laboratory. Therefore, findings and performance in laboratories cannot be extrapolated to an industrial fermenter. The variations/gradients in the fermentation broth during the fermentation processes affect the metabolism of the biological agent (organism). This may result in a reduction of its performance, and consequently, the bioprocess is driven away from the conditions for yield/productivity optimization. Therefore, it is crucial to understand the metabolism changes relating to the biological agents to optimise the fermentation process.

Traditional bioprocess modelling has largely made use of either empirical/ab-initio, or the more complex first principles modelling. However, these approaches assume reactions and inputs to be ideal or substances to be at an equilibrium thermodynamic state throughout the bioprocess. Furthermore, they model the process as a whole, and thus fail to include system-level and component-level complexity into account.

The microorganism' metabolism changes involve complex dynamic intra-cellular and extra-cellular interactions, both in their local and global cellular environments. It is extremely difficult to obtain detailed experimental insight into local fermentation environment which spans a large scale, typical of an industrial reactor. Thus studies have concentrated on equipment-scale measurements which may suffice for laboratory set-ups. However, availability of information on a heterogeneous fermentation environment as experienced by the microorganism is a requisite for industrial scale reactors. Sensors in a bioreactor may provide temporal information, but no spatial information or only very limited spatial information.

In bioprocesses, even within a certain population of microorganisms, there are cellular subpopulations with different metabolic reactions and fluctuations in their rates. Such variations have a major impact on productivity and yield of bioprocesses. Furthermore, the microorganism continuously observes spatial and/or temporal changes in its local environment during the process. An accurate model would therefore also need to take into account the historic data of the interactions/changes observed by the microorganisms, and their population variation inside the fermenter over time. To accurately study and model real life industrial bioprocesses, it is of paramount importance to not only model reactant mixing in bioreactors/fermenters, in particular, to model the variation in feed component/substrate composition within a reactor, but also to model the microorganisms themselves, their lifeline over time, and to record substrate fluctuations from their perspective. There are however computational restraints on the amount of data which can be processed and thus taken into account in mathematical modelling of such complex environments. Furthermore, it is not clear in art if and how such modelling may be used to control performance of industrial bioprocesses.

Pathi (2005) Mathematical Modeling of Transport and Reaction in Cellular and Tissue Engineering, relates to mathematical modeling of transport and reaction in cellular and tissue engineering. Chapter 6 relates to a study intended to enhance understanding of various factors that affect cell growth and ECM production *in vitro* in bioreactors. The model would enable a further rationalization of experimental results providing suggestions for improved experimental design.

WO2018/229802 relates to a method for predicting outcome of and modelling of a process used for manufacturing a sample intended to be used in another system. The problem to be solved is to develop a procedure for creating process models than can serve as a digital representation of a process and used for manufacturing a sample in a biological system, such as a bioreactor. This is solved by combining historic data and current data.

There is thus a need in the art to implement a system and method for monitoring and/or controlling an industrial bioprocess, like fermentation in an industrial fermenter, that makes use of a dynamic model of the bio (fermentation) process as seen through the frame of reference of the microorganism, and that puts relatively less burden on computational resources. More specifically, a system and method that provides an effective way to make decisions, manual or automated, to optimize performance of the fermentation process over time, to predict the course of the process as well as to adapt to changing conditions is needed.

In the context of modelling, a digital twin (DT) may be configured to simulate operation of an asset such as a machine (hardware), an equipment, a software process, and the like. For example, a DT may represent a digital copy of the asset, created in a manner which replicates the real world system in form and behaviour. In addition to generating a simulated representation, the digital twin can also be configured to communicate to a user or another machine via different communication channels. Furthermore, the digital twin may be configured to communicate with other digital twins in order to form an integrated network of twins which simulate operations of different assets of at least one process or at least one product.

Assets may be provided with one or more sensors, or measurement sensors, configured to monitor different operations and/or conditions of the asset, and the environment in which the asset operates. With the advent of the industrial Internet of Things (IloT), data from such sensors and other data sources, can be recorded or transmitted to a cloud-based computing environment, or another distributed computing resource which can be located remotely from the asset. The measured and/or processed data can then be fed to the DT to improve its working. The cloud computing system can further include or can be coupled with one or more processors to perform a specific task related to asset maintenance, analytics, data storage or some other function. Through the use of such a DT, a manufacturer of industrial based assets, for example, an industrial bioreactor, can be uniquely disposed to improve its understanding of assets themselves, models of such assets, and industrial operations involving these assets. However, a DT of an industrial reactor (asset) can only control an industrial bioprocess (operation relating to the asset) reliably if the constraints that have gone into realizing the DT represent a near-perfect simulation of the industrial fermentation environment.

### Summary

The present invention is directed to solving at least the technical problems and disadvantages mentioned in the above section.

According to an aspect of the invention, a computer-implemented method for monitoring and/or controlling performance of an industrial bioprocess comprising the steps of:
providing a first model incorporating spatial variation of a process parameter in a bioreactor during the bioprocess;
providing a second model which incorporates the local response of a microorganism to the process parameter observed by said microorganism;
generating a digital twin, DT, of the bioreactor by combining the first model and the second model; wherein an output of the first model is an input to the second model and/or wherein an output of the second model is an input for the first model;
providing the DT with an input operational setting;
predicting, by the DT, a characteristic of the bioprocess based on the provided input operational setting;
monitoring and/or controlling the bioprocess based on the predicted characteristic of the bioprocess.

In a preferred embodiment, the predicted characteristic of the bioprocess comprises a predicted process parameter and/or a predicted process performance.

In a preferred embodiment, the present step of predicting, by the DT, a characteristic of the bioprocess comprises predicting the characteristic of the bioprocess for more than one input operational setting.

In another preferred embodiment, the present bioprocess is controlled by tuning the input operational setting based on the predicted characteristic of the bioprocess.

In a preferred embodiment, the first model comprises a hydrodynamic model which calculates a spatial variation in at least one process parameter, preferably the concentration of a substrate like sugar or dissolved oxygen, the shear rate, pH or the temperature at a predetermined location inside the bioreactor.

In a further preferred embodiment, the second model comprises a metabolic model which describes a state of the microorganism in the bioreactor.

In a preferred embodiment, the present first model and second model are combined directly, or wherein the first and second model are combined in a segregated manner.

In a preferred embodiment, the method comprises measuring the input operational setting and/or process parameter via at least one measurement sensor.

In a preferred embodiment further, the present method comprises feeding the DT with the predicted characteristic of the bioprocess.

In a preferred embodiment the method further comprises:
determining a deviation between the predicted characteristic and a measured characteristic of the bioprocess;
if the deviation is greater than a threshold, determining an offset in the bioprocess; signalling the offset in the bioprocess.

In a preferred embodiment, the method further comprises:
providing the DT with a current time evolution and alternative time evolution of the input operational setting;
predicting a time evolution of the predicted characteristic of the bioprocess based on provided time evolutions,
determining whether the time evolution of the predicted characteristic of the bioprocess is better for an alternative than the current time evolution;
tuning the operational setting based on the determination.

In a preferred embodiment the method further comprises:
detecting a process upset based on a deviation between a measured process parameter and/or operational setting and the predicted characteristic of the bioprocess;
calculating an impact of the process upset on the predicted characteristic of the bioprocess;
determining to control the bioprocess based on the calculated impact of the process upset, preferably to continue or discontinue the bioprocess.

In a preferred embodiment the predicted process parameter comprises at least one of substrate concentration, pH, temperature, dissolved oxygen, dissolved CO₂, microorganism concentration, (by)product concentration, shear stress, gas bubble size, viscosity, gas liquid mass transfer coefficient, rheology, morphology of microorganism, intracellular concentration of pools (enzymes & metabolites), local heat transfer, mass transfer, volume of broth and total volume in the bioreactor during the bioprocess.

In a preferred embodiment, the predicted process performance comprises at least one of energy usage of the bioprocess, ethanol production, and yield of a product of interest.

In a preferred embodiment, the measurement sensors comprises at least one of temperature, pressure, gas, mass analyser, velocity and pH sensors.

In a preferred embodiment, the input operational setting comprises at least one of inflow of substrate, inflow of air, air composition, outflow of gas, operating temperature or cooling flow, stirring speed, inflow of acid/base, and operating pressure.

According to another aspect of the invention, the present invention relates to a digital twin of a bioreactor for online monitoring and/or control of an industrial bioprocess comprising:
a first compartmental model incorporating spatial variation of a process parameter in a bioreactor during the bioprocess; and
a second model which incorporates the local response of a microorganism to the process parameter observed by said microorganism, wherein the digital twin is configured to performed the present method steps.

In a preferred embodiment, the first model is a compartmental computational fluid mechanics, CFD model.

According to yet another aspect of the invention, device for monitoring/and controlling an industrial bioprocess is provided, wherein a controller of the device is configured to execute any of the method steps of the appended claims.

According to yet another aspect of the invention, a computer program product comprising instructions is provided, which, when the program is executed by a computer, causes the computer to carry out any of the method steps of the appended claims.

Before undertaking the detailed description below, it may be advantageous to set forth definitions of certain words and phrases used throughout this patent document: the terms "include" and "comprise," as well as derivatives thereof, mean inclusion without limitation; the term "or," is inclusive, meaning and/or; the phrases "associated with" and "associated therewith," as well as derivatives thereof, may mean to include, be included within, interconnect with, contain, be contained within, connect to or with, couple to or with, be communicable with, cooperate with, interleave, juxtapose, be proximate to, be bound to or with, have, have a property of, or the like; and the term "controller" means any device, system or part thereof that controls at least one operation, such a device may be implemented in hardware, firmware or software, or some combination of at least two of the same. It should be noted that the functionality associated with any particular controller may be centralized or distributed, whether locally or remotely. Definitions for certain words and phrases are provided throughout the description, those of ordinary skill in the art should understand that in many, if not most instances, such definitions apply to prior, as well as future uses of such defined words and phrases.

### Figures

For a more complete understanding of the present disclosure and its advantages, reference is now made to the following description taken in conjunction with the accompanying drawings, in which like reference numerals represent like parts. The drawings described herein are for illustration purposes only and are not intended to limit the scope of the present disclosure in any way. The scope of the invention is defined by the claims.
Figure 1 illustrates a typical, prior art fermentation process and the components involved therein.
Figure 2 exemplifies a model to study the variation in feed component/substrate composition within a reactor according an embodiment of the invention.
Figure 3 shows a fermenter according to an embodiment of the invention. (Production fermenter)
Figures 4a, 4b show the formation of compartments in the fermenter according to an embodiment.
Figures 5a, 5b illustrate a metabolic model of a particle micro-organism, according to an embodiment of the invention.
Figures 6a and 6b illustrate generation and operation of a digital twin for process monitoring in an industrial reactor, according to an embodiment of the invention.
Figure 7 shows a control of an industrial operation using the aforementioned DT, according to an embodiment of the invention.
Figure 8 illustrates verification of the digital twin for process monitoring in an industrial reactor, according to an embodiment of the invention
Figures 9a and 9b show a method of using the digital twin for online process optimization in an industrial reactor, according to an embodiment of the invention.
Figures 10a and 10b illustrate a method of using the digital twin for online process optimization in an industrial reactor, according to yet another embodiment of the invention.
Figure 11 shows a device which incorporates the DT of the present invention.
Figure 12 shows a schematic setup for controlling an industrial bioprocess using the DT of the present invention.

### Detailed description

The following description with reference to the accompanying drawings is provided to assist in a comprehensive understanding of exemplary embodiments of the invention as defined by the claims. It includes various specific details to assist in that understanding but these are to be regarded as merely exemplary. Accordingly, those of ordinary skill in the art will recognize that various changes and modifications of the embodiments described herein can be made without departing from the scope of the invention. In addition, descriptions of well-known functions and constructions may be omitted for clarity and conciseness.

The terms and words used in the following description and claims are not limited to the bibliographical meanings, but, are merely used by the inventor to enable a clear and consistent understanding of the invention. Accordingly, it should be apparent to those skilled in the art that the following description of exemplary embodiments of the present invention is provided for illustration purpose only and not for the purpose of limiting the invention as defined by the appended claims.

Unless defined differently, all terms used herein, which include technical terminologies or scientific terminologies, have the same meaning as a person skilled in the art to which the present disclosure belongs. Such terms as those defined in a generally used dictionary are to be interpreted to have the meanings equal to the contextual meanings in the relevant field of art, and are not to be interpreted to have ideal or excessively formal meanings unless clearly defined in the present disclosure. Although reference has been made to an industrial bioprocess for sake of illustration of application of the method, it is clear to the skilled person that the method and/or the product is equally suitable for use in any other similar industrial process.

A bioprocess is defined as cultivating a microorganism or biological agent to obtain a product of interest. In other words, the bioprocess is a fermentation process.

A substrate is the material that is utilized during the bioprocess, such as oxygen, sugar etc. The substrate is utilized by the microorganism.

A microorganism may be any microorganism, including single cells like single cell proteins. Preferred microorganisms are bacteria, like lactic acid bacteria, yeast, like *Saccharomyces, Kluyveromyces* or *Torula,* or *Yarrowia,* and fungi like filamentous fungi. Examples of suitable fungi are *Aspergillus* species, like *Aspergillus niger* and *Aspergillus oryzae.*

A product of interest as defined herein is a product produced by cultivating the microorganism. For example, the product of interest is an enzyme, a metabolite, a sweetener, a vitamin or a colorant.

A grid cell may be considered as a region of space in a spatial model for which the process parameters are computed. The process parameters may be computed using a set of equations in the model.

The present disclosure aims to provide a more accurate digital representation of the fermenter and the fermentation process, by constructing a digital twin which takes into account not only reaction dynamics in a fermenter but also metabolism of the microorganism due to said reaction dynamics. The present disclosure further aims to couple the industrial bioreactor dynamics to digital twin technology, in order to improve the bioprocess. A characteristic feature of the method and apparatus thereof is that it makes use of a model which relates the individual processing equipment (the asset) of the industrial process, the fermenter, to the process parameters of the fermentation process. The model can further be used to monitor an industrial bioprocess, so as to predict whether the bioprocess yields products of optimal quality and/or quantity. It is further an object of the invention to implement the model so as to predict an optimal operational strategy for the industrial process, and to signal alerts if a deviation is observed in the process, to further assess how changes in operation of the fermenter/ asset affect the quality and quantity of the product.

Figure 2 exemplifies a model to study hydrodynamics of feed components within a reactor according an embodiment of the invention. In particular, figure 2 shows a fermenter 200 and a simulation of how mixing takes place inside the fermenter/reactor during a bioprocess. Figure 2a shows a mixing simulation at the highest resolution. Figure 2b shows a compartmentalized simulation which is derived based on the simulation in figure 2a. Figure 2c shows an ideal mixing state of the substrate inside the fermenter.

In an embodiment, the first model incorporating spatial variation of a process parameter in a bioreactor during the bioprocess, is a compartment model, a CFD model, a recurrence CFD model and/or a neural network based model.

In an embodiment, the second model incorporates a local response of a microorganism to the process parameter observed by said microorganism by calculating local process parameters. Preferably, the second model comprises a metabolic model which describes a state of the microorganism in the bioreactor.

In an embodiment the present process parameter, or present predicted process parameter, is chosen from substrate concentration, pH, temperature, dissolved oxygen, dissolved CO₂, microorganism concentration, (by)product concentration, shear stress, gas bubble size, viscosity, gas liquid mass transfer coefficient, rheology, morphology of microorganism, intracellular concentration of pools (enzymes & metabolites), local heat transfer, mass transfer, volume of broth and total volume in the bioreactor.

In an industrial bioprocess, an ideal mixing assumption (e.g. Monod kinetics), although computationally of advantage, leads to inaccuracies in calculation, due to large-scale substrate gradients in the bioreactor. The microorganisms, from their frame of reference, experience strong temporal and/or spatial variations in the substrate concentration, as they move across different locations in the bioreactor. Such gradients not only occur in substrate concentrations, but also in other process parameters like dissolved oxygen (DO), pH, temperature etc.

According to an embodiment of the invention, a computation fluid dynamics (CFD) may be used to model hydrodynamics (circulation velocities, mixing, e.g. turbulent mixing), and to study environmental fluctuations from the microbial point of view by registering their state across time. While the model gives a detailed insight into the fermentation environment with high resolution, experimental studies are tedious and are mostly limited to laboratory set-ups, which do not represent the mixing environment in an industrial scale bioreactor.

CFD calculations to study the reactant mixing in a bioreactor may be implemented as ideal, compartmental or full CFD models. An ideal model assumes a perfect mixing of reactants inside the bioreactor. Although limited by ideal assumptions, this model requires the least amount of computational resources among all CFD models.

A full CFD computation provides the most reliable optimization of mixing in the bioreactor, taking into consideration the spatial variation of substrate concentration in the reactor. Figure 2a shows a schematic full-CFD representation of substrate concentration/concentration gradient at a certain time T in an ongoing bioprocess. If 201 is considered to be a central axis of the fermenter 200 in its longitudinal direction, the computation yields a strong heterogeneity in the substrate concentration due to mixing in longitudinal/and transverse directions with respect to the axis. This is particularly noticeable at/or near the spatial locations of impellers 202, the impellers 202 being situated on the central axis 201. Impellers are agitators whose movement helps towards continuous stirring of media, distribution of oxygen throughout the system and preventing cells from settling down. They may be rotary-fan shaped, each impeller comprising, for example, 3 blades located equidistant from one another, and on the central longitudinal axis 201 of the fermenter 200. The impellers may further be distributed on the axis at predetermined height intervals defined on the central axis, with a certain clearance from the bottom of the fermenter.

A full-CFD calculation however, comes at the cost of computational power, and multi-impeller systems produce a more complicated flow pattern. For example, in a fermenter with 3 impellers, each impeller generates an independent flow pattern, which needs to be taken into account for the computation. This is further complicated in a fermenter in which air is introduced, as in the case of the present invention. As may be understood from figure 2a, different regimes of flow pattern, and/or substrate concentrations may exist in the reactor based on the intensity of mixing by the impeller and/or intensity of aeration.

To improve the computational efficiency of full/direct CFD simulation and simultaneously reproduce the spatial heterogeneity inside the bioreactor, an embodiment of the invention uses a CFD based compartment model, which bases its model on the flow pattern and/or substrate gradient observed in the full-CFD. The compartmental model divides the spatial domain of the fermenter into a limited number of interconnected volumes or compartments. Each compartment may further comprise gridcells in which the flow properties (turbulence, concentration, etc.) are assumed homogeneous. Quantities such as particle velocity, gas fraction, oxygen concentration, sugar concentration, etc. are defined for each gridcell.

The compartmental models in the present invention are developed based on the CFD results while solving the turbulent liquid flow using full CFD, for example, based on the particle velocity profiles obtained using CFD. In the compartment model, each circulation loop revealed e.g. by the velocity profiles of the CFD simulation may be considered as one compartment. This is based on the fact, that mixing is very fast in each circulation loop and hence can be regarded as a well-mixed regime. Each gridcell may be considered a sub-unit of a compartment. The use of the output of full CFD is aimed to improve the accuracy of the traditional compartmental simulations where fluxes between the compartments are deduced from global parameters like the flow number. From the definition of the compartments/zones and of the flow rates between them, the time and/or spatial evolution of the e.g. concentration distribution of a substrate and thus of its mixing can be simulated using differential equations. Compartment models provide satisfactory accuracy while requiring significantly less resources, even with complex biological processes involved. A compartment model makes it especially useful for its application to online process applications, which is too time-consuming for a full CFD model. To verify the compartment model, simulation results may be validated by comparison with laboratory experimental data.

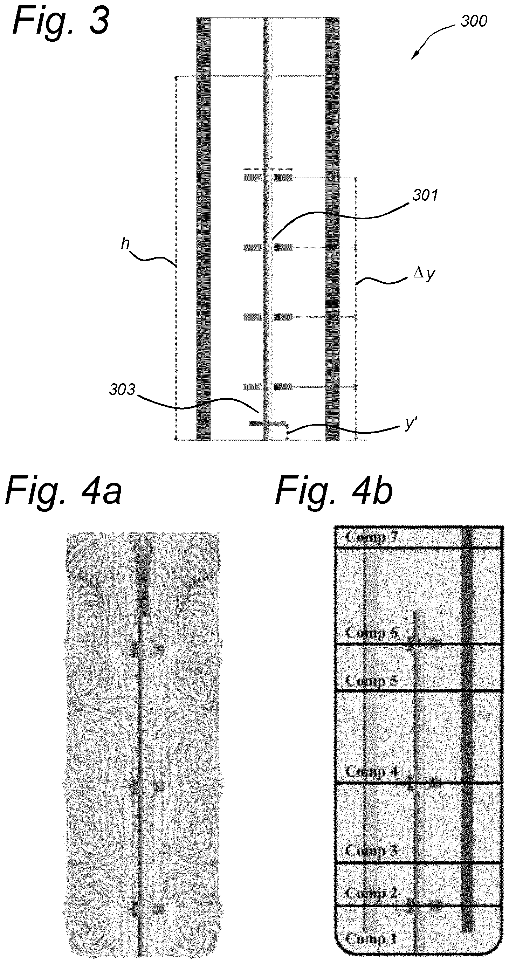

Figure 3 illustrates a setup of a fermenter/bioreactor, whose results are used for the development and/or validation of the CFD model, according to an embodiment of the invention. The bioreactor used for the development of the hydrodynamic model is a fermenter 300 having a volume of 30m³. The fermenter has a diameter of 2.09m and a liquid filled height of 6.55m, and comprising at least one impeller 301. As shown in figure 3a, the impellers may further be distributed on the axis (y) 302 at predetermined height intervals delta y, with a certain clearance y' from the bottom of the fermenter. There may be other examples of how impellers may be located in a bioreactor. A predetermined agitation rate may further be set. The reactor of the present invention may further comprise an aerator or a sparger (303) for introducing sterile air or oxygen to the media in the fermentation process. The sparger may be introduced in various shapes and located at a predetermined height within the vessel. Gas is fed via the sparger with a fixed or variable gas flow rate. Experimental studies may further be initiated using fermenter 300 using techniques like Laser Doppler Anemometry (LDA), Particle Image Velocimetry (PIV), to quantify the flow pattern and substrate concentration within the reactor.

Figure 4a shows, using the geometry of the laboratory fermenter, the results of the CFD simulation. In particular, it reveals a cross-section of a plurality of circulation loops formed, e.g. by the velocity profiles of at least one particle inside the fermenter. A plurality of loops formed on a transverse side of (in a perpendicular direction to) the central axis and along a same vertical (y) offset from the bottom of the fermenter may be considered as one compartment. This is shown in figure 4b. The compartmentalisation is based on the principle that mixing is very fast in each circulation loop and hence can be regarded as a well-mixed regime.

In an embodiment of the invention, for turbulence modelling in fermenters, Reynolds Averaged Navier Stokes (RANS) methods may be used. They model the effect of turbulence, while determining the global flow patterns and concentration gradients within the bioreactor. The CFD simulation may be implemented using a standard k-e (kinetic energy-energy dissipation rate) turbulence model and multiple reference frame (MRF) model to simulate the impeller motion. The simulation may be carried out using commercial software like Fluent, e.g. Ansys Fluent.

According to an embodiment of the invention, inter-compartment particle mass exchange is further modelled in a multi-impeller vessel. Transfer of mass may occur due to removal of carbon dioxide from the cells, conversion of oxygen from the gas phase to the liquid phase, release of sugar monomers and their conversion from solid to liquid phase etc.

As aforementioned, the present CFD model enables the prediction of local flow profiles, and in turn, local distributions of substrate, dissolved oxygen and other extracellular parameters which influence the micro-organisms.

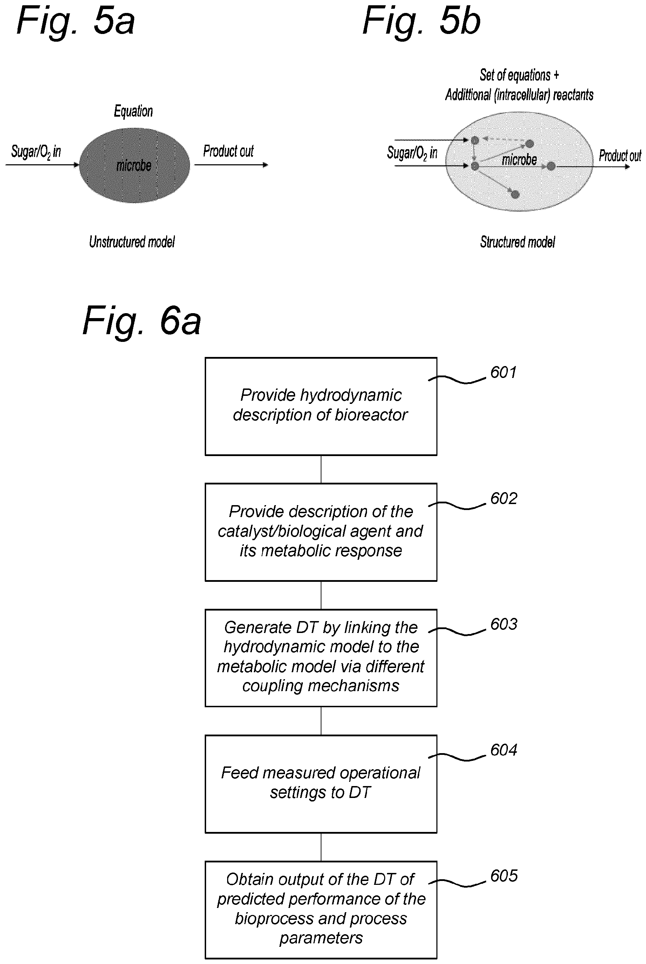

The CFD model may further be supplemented with information on the biological reactions which take place inside the bioreactor during a bioprocess. This is in turn used for prediction of the bioprocess parameters and/or process performance. Figures 5a and 5b detail how biological reactions can be modelled within a bioreactor during a bioprocess.

According to an embodiment of the invention, biological reactions may be studied with unstructured kinetic models, which simulate only the uptake of the substrate, assuming an instantaneous adaptation of the metabolism to the extra cellular conditions. As shown in figure 5a, the unstructured model directly links substrate (sugar, oxygen) input/uptake rate to the output/product growth, e.g. via a Herbert-Pirt equation. Minimal details on the intracellular reactants are taken into account in such a model. Further, the model bases itself on cell performance under ideal/equilibrium conditions assuming there is no spatial variation in of process parameters in the bioreactor. The model has limited capability to cope with changes in cellular conditions, for example, when the intra-and extra-cellular conditions are not in equilibrium, however, is especially useful to maintain computational costs at bay, and still be able to obtain a reasonable simulation of the amount of substrate uptake in a bioreactor, and consequently, obtain a dependence of the kinetic parameters on the reaction conditions.

According to an embodiment of the invention, the metabolism of the microorganism may be modelled using a structured kinetic model. A structured reaction model is a model of the microorganism that includes a state description of the microorganism based on one or more components. This is shown in figure 5b. State is a concentration or an activity. For a microorganism, these are membrane-bound or intra-cellular components/reactants, e.g. a concentration of metabolite, concentration / activity of enzyme, concentration of RNA, DNA, protein, lipid, etc. It is well-known to a skilled person that the bioprocesses may not be limited to the use of organic microorganisms like microbes, but also, for example, to heterogeneous inorganic microorganisms. In case of a heterogeneous inorganic microorganism, said components may be surface sites of the microorganism particle as well as species bound to the microorganism surface. For example, the component may be a contaminant (sulphurous compound, nitrous compound, oxygenated compound, carbonated compound, char,...), or a bound substrate/intermediate/product (organic or inorganic compound). In a structured kinetic model, the micro-organisms are modelled as a number of virtual particles (parcels) carrying an internal parameter vector describing their state. A number of parcels inside a cell and their mutual interactions can be tracked for each parcel. The use of parcels helps study the observed extra-cellular conditions over time for each parcel, and to monitor the intra-cellular response to these conditions.

According to an embodiment of the invention, different coupling mechanisms may be employed to couple biological reactions to CFD simulations, e.g. via Eulerian models, population balance models (PBM) or Euler-Lagrange models, and thus determine the reaction rates in a bioprocess based on the impact of spatial and temporal variations of process parameters on the reaction rate.

The microorganism sees a continuous change in substrate concentrations (extra-cellular changes) as they move around, spatially and/or temporally. The extra-cellular changes or interactions further influence the intra-cellular state of the microorganism. Both PBM and Euler-Lagrange models can account for adaptation of the metabolism of the microorganism to environmental fluctuations. The microbes are modelled as a component of the liquid phase, or, in other words, separate from the liquid. In PBM, the biomass specific growth rate (µ) is applied to describe population heterogeneity, using empirical relations to specify how the growth rate (µ) adapts to the local extracellular environment. The model solely considers the growth rate adaptation as a function in determining the reaction rates.

Metabolic fluctuations may take place on shorter timescales than growth rate fluctuations. To account for this, according to a preferred embodiment of the invention, particle-based (Lagrangian) simulations coupled with structured metabolic models may be employed to track micro-organisms and record substrate variations from their frame of reference. From the spatial gradients observed from the organism's frame of reference, it is then possible to construct 'parameter versus time' series for each parcel, which gives insight into at least the growth rate and the substrate uptake to extra-cellular variations and distributions of the magnitude and duration of extra-cellular variations. Thus, the coupling takes into account the history of the particle's extracellular exposure, which also influences its intracellular state. It is emphasized that although reference is made to a particle, the number of particles considered in the Lagrangian modelling is at least one, with no maximum.

The hydrodynamic model calculates, for example, the local liquid-phase species concentrations as a result of mixing and uptake by the microorganism. The metabolic model calculates what said uptake rate of the microorganism is, based on the local species concentration. Hence, preferably the output of the hydrodynamic model (the species concentration) serves as input to the metabolic model, and vice-versa.

The models may be coupled directly to each other using a suitable numerical method e.g., via a coupled solving algorithm. The result/output of the coupling is then rendered simultaneously. A segregated solving approach may also be used, wherein the numerical model first solves one model, and sequentially the second, and iterates this loop until a solution is reached. The iterations can either be carried out within a single time step (iterate a few times before moving to the next time step), or within iterated time steps (one iteration per time step).

Using the aforementioned models, the hydrodynamics of feed components within a reactor and the metabolism of the microorganism affected by such hydrodynamics are coupled to each other, which in turn may be used to conceive a DT in virtual space. The DT can be configured to monitor and control the bioprocess in real time.

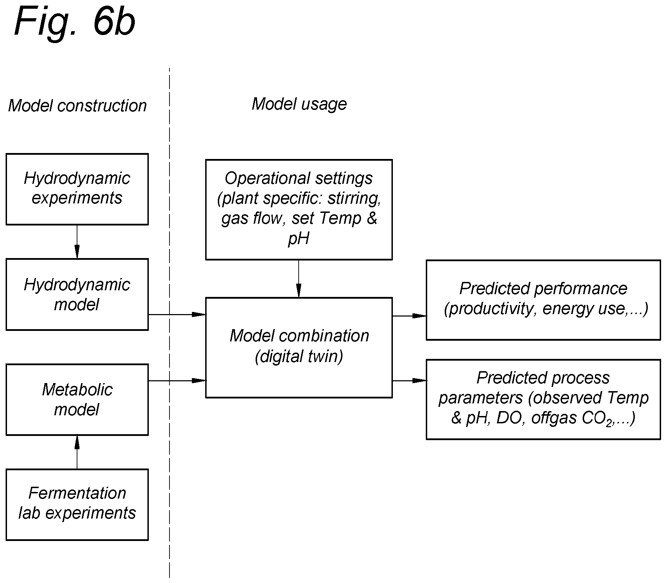

Figure 6a shows a flowchart illustrating generation and operation of a digital twin for process monitoring in an industrial reactor, according to an embodiment of the invention. Figure 6b is a block diagram illustrating the same.

Step 601 involves providing a hydrodynamic description of the bioreactor, by at least one of the aforementioned CFD models. The CFD models may comprise a compartmental CFD model, full-CFD model, CFD model, rCFD model, and tanks-in-series model. The generation of the hydrodynamic model may further comprise validation of the generated model using experimental data, correlation studies, empirical relations or first-principles methods such as CFD.

Step 602 involves providing a description of the microorganism/biological agent and its metabolic response to cellular environments for use in the hydrodynamic model. As indicated above, the metabolic model may be structured and/or unstructured. The metabolic model may further be verified using experimental fermentation results.

Step 603 involves linking the hydrodynamic model to the metabolic model via different coupling mechanisms. A preferred embodiment couples the two models using a Lagrangian descriptor. Thus, the DT of the fermenter is generated by combining the hydrodynamic model with the unstructured or structured metabolic model. The generation of the DT may further comprise generation of a DT interface to manage the bio/fermentation process. A digital equivalent of the fermenter may include process information relating to the bioprocess as well as a digital representation of the fermenter. The step may further include displaying, via a user-interface, the DT representation of the fermenter.

Step 604 comprises feeding the operational settings measured by these sensors to the generated DT model, the operational settings comprising an industrial plant specific process parameters like inflow of substrate, inflow of air, air composition, outflow of gas, operating temperature or cooling flow, stirring speed, inflow of acid/base, operating pressure, etc. The process parameters are herein referred to as operational parameters or operational settings of the bioreactor. Depending on the operational setting/process parameter to be measured, a plurality of different sensors may be installed in the bioreactor. For example, the plurality of sensors may include temperature sensors, pH sensors, gas sensors, pressure sensors, and the like.

The operational settings may further be set using data from a real process plant (fermenter). Thus, according to an embodiment of an invention, there exists a fermenter in real space different from the asset used to generate the models and providing data to a DT in a virtual space. The output of the DT may be used to monitor a real process, i.e., output data from the DT may be compared to the real process. If unexpected deviations occur between the DT output and real process parameters, an operator may be informed by suitable reports / alarms. Alternatively or additionally, such DT output may further be used to optimise the real space in a feedback loop, in other words, the DT in the virtual space may also provide data to the real space.

As mentioned above, a state of a particle may be described using an internal parameter/component vector. The values of this component vector for each Lagrangian particle, considered in the Lagrangian tracking, can be used to describe the overall state of the microorganism. The state of the reactor is described by the value of model process parameters (e.g. substrate uptake, gas fraction, oxygen concentration, sugar concentration) at each location.

Step 605 comprises the DT providing an output by predicting performance of the bioprocess and the process parameters. As an example of predicting the process parameters, the DT calculates, at a time T1, the state of the particle (e.g. growth rate, intracellular concentration of pools) and state of the bioreactor (e.g. substrate concentration, pH, temperature, dissolved oxygen, dissolved CO₂, microorganism concentration, (by)product concentration, shear stress, gas bubble size, viscosity, gas liquid mass transfer coefficient, rheology, morphology of microorganism, intracellular concentration of pools (enzymes & metabolites), local heat transfer, mass transfer, volume of broth and total volume in the bioreactor). The DT may further be input with operational settings of the plant or the state of the reactor measured in real space. On the basis of the fed operational settings and the state of the microorganism (microbe) and/or the reactor at time T1, the DT computes the state of each tracked particle/microorganism and the reactor at a later time T2.

The performance of a process is determined by indicators like yield or conversion efficiency, productivity or rate, titer, quality and energy usage of the process. The metabolic model can calculate concentrations, rates and/or heat production which are indicators of the productivity of the process, which is common knowledge for the person skilled in the art. The DT interface may output the predicted parameters, together with the set/operational parameters.

Through the use of such a DT, a manufacturer of an industrial bioreactor (asset) can be uniquely disposed to improve its understanding of the bioreactors themselves and industrial operations (bioprocesses) involving these bioreactors.

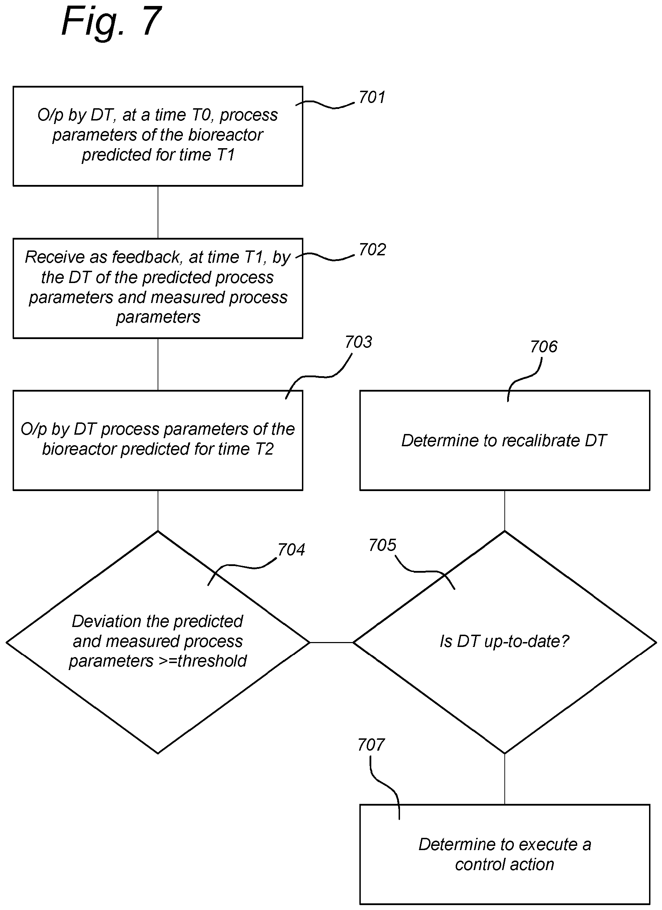

Figure 7 shows a control of an industrial operation using the aforementioned DT, according to an embodiment of the invention. In doing so, the method relates the DT of the individual processing equipment (bioreactor) to the performance parameters of the overall bioprocess.

Step 701 comprises predicting by the generated DT, at a time T0, process parameters of the bioreactor for time T1. For example, the DT may predict a value of at least one of substrate concentration, pH, temperature, dissolved oxygen, dissolved CO₂, microorganism concentration, (by)product concentration, shear stress, gas bubble size, viscosity, gas liquid mass transfer coefficient, rheology, morphology of microorganism, intracellular concentration of pools (enzymes & metabolites), local heat transfer, mass transfer, volume of broth and total volume in the bioreactor during the bioprocess. The DT may be generated using the method described above, and will not be discussed again, for sake of brevity.

Step 702 comprises the DT receiving as feedback, at time T1, the predicted process parameters. The step may further comprise receiving the measured operational settings by the DT and/or measured process parameters. The operational settings and/or process paramters may be measured real-time (at T1) by the sensors.

Step 703 comprises predicting, by the DT, process parameters of the bioreactor (state of the bioreactor) which are existent at time T2.

Step 704 comprises comparing, by the DT, the predicted process parameters for time T2 with the measured process parameters and/or measured operational settings at time T2. As mentioned above, these parameters may be those measured by the one or more sensors equipped inside (or outside) the fermenter (plant), and may correspond to the measured values of at least one of dissolved oxygen, substrate concentration measurements, temperature, pH, offgas composition, velocity measurement, osmolarity etc. Both the predicted and the measured state of the bioreactor (process parameters) may be displayed to the operator, and/or fed as input to another machine/processor. It is then determined whether there is a difference observed between the prediction by the DT in the virtual space and the observed parameters in the real space, and/or whether the deviation exceeds a predetermined threshold which indicated acceptable deviation. If there is no deviation, or if the deviation is below the acceptable predetermined threshold, there needs no control action to be undertaken. This helps to optimise correction procedures to be carried out in the asset (fermenter), while keeping false alarms or unnecessary maintenance/ correction at bay.

Step 705 comprises, if there is a deviation between the predicted and observed/measured process parameters and/or operational setting, the DT determining the source of the deviation, as will be explained in more detail hereinafter.

It is verified by the DT whether the deviation is caused by an issue in the model used to generate the DT. Once established that the error lies in its generation, a model recalibration is suggested to be performed in step 706. A calibration/verification procedure of the DT may be undertaken offline or online. Alternately, a periodic calibration may be undertaken to ensure that the model is accurate.

If it is established that there is indeed a deviation between the predicted and measured values while using a verified DT, the DT determines to execute a control action in step 707. A control action may comprise an automatic adjustment or a human input based adjustment of operational setting, the display of a notification message, e.g. flashing or sounding of a warning message for the operator, etc. The particular control action which may be implemented to signal process parameter deviation is not limited to any means.

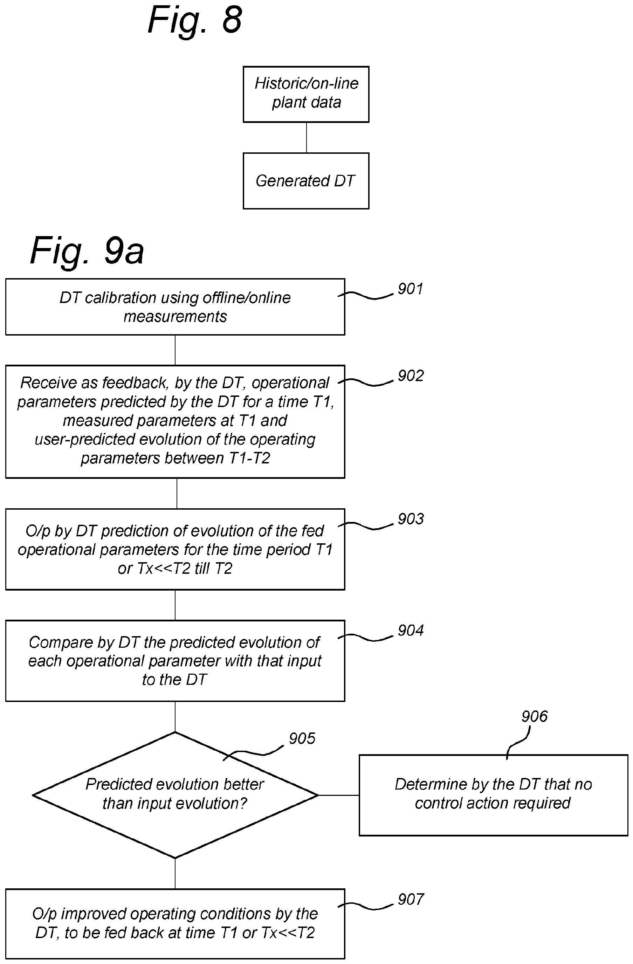

Figure 8 shows different methods of verification that can be applied to the DT. It is optional, and sometimes desirable, to verify performance of the DT before using it in a bioprocess to predict the process parameters. In other embodiments, this may be undertaken to simply ensure that the DT is consistent with a condition of the bioreactor/fermenter as it exists in reality.

The verification can be performed offline, based on historic plant data supplied from a real space. The real space may, for example, be the current industrial plant, or a neighbouring plant with similar operating conditions. It is determined whether the predicted process performance and process parameters match the recorded/measured process parameters and performance. As an example, it may be compared whether a measured yield of a substance [=productivity] matches that predicted by the DT.

According to another embodiment of the invention, the verification may be performed online, using online plant data. The process parameters are monitored real-time by the sensors equipped in the bioreactor. The bioreactor may be equipped with any number and kind of sensors to obtain readings of the parameters. The sensors may be comprised of, but are not limited to, temperature sensors, pH sensors and oxygen sensors. An online verification is especially useful to assess whether the DT not only assesses the observed process performance, but whether it can also be adjusted real-time to process variations.

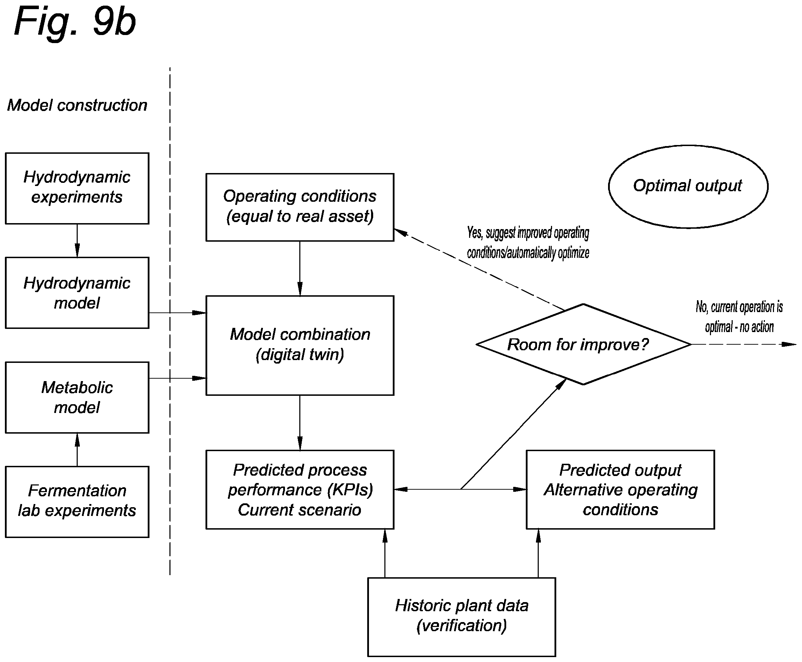

Figure 9a shows a method to predict future performance/online process optimization of the bioreactor according to an embodiment of the invention. Figure 9b shows a corresponding block diagram.

Step 901 comprises a calibration of the DT based on measured operational settings/parameters.

Step 902 comprises feeding the DT with predicted operational parameters or predicted characteristics of the bioprocess by the DT for a time T1, and those measured real-time by the sensors. The former may be implemented via feedback mechanism, wherein the DT receives as feedback at a certain instant of time, the parameters predicted by the DT for that time instant. The step also comprises providing the DT with, or the DT receiving, a description of evolution of the fed/input operational parameters between time T1 and T2. Time T2 may lie significantly ahead of T1.For example, the temporal separation between points T1 and T2 may lie in the order of days, weeks or months. With the description of evolution of each operational parameter, the DT receives a current operation strategy of the bioreactor pertaining to the parameter. An operational strategy may comprise information of how the operational parameters evolve over a course of time from the start of the fermentation process to its completion. For example, if the operating parameter relates to inflow of substrate into the bioreactor, the operational strategy may indicate the course of the inflow over a period of time. The estimation of evolution of the operating parameter may be based on values input manually by an operator, for example, based on historic plant data, or based on data generated by a suitable algorithm.

In step 903, the DT predicts at time Tx, where T1 <Tx<T2, an evolution of the fed/received operational parameters for the time period Tx till T2. In case of a real-time prediction, the DT outputs an evolution of the operational parameters at T1 for the period, i.e. T1-T2.

From each predicted evolution of an operational parameter by the DT, a process performance (evolution of the state of the process) may further be predicted. For example, based on the predicted evolution of the inflow of the substrate between time Tx and T2, or between T1 and T2, a (future) performance of the process may be predicted. The process performance may be quantified by calculating values for key performance indicators such as, for example, biomass composition, total biomass amount, max. yield, minimal formation of key by-products, minimal energy use, etc.

Each predicted evolution of each operational parameter represents a prospective alternate operational strategy/control scenario to run the fermentation process in the bioreactor. Hence, the method simulates multiple control scenarios which can be analysed and possibly used to run the bioprocess.

Step 904 comprises the DT comparing the predicted operational strategy with respect to each operational parameter with the corresponding current strategy input to the DT, and step 905, determining whether the prediction of the process performance by the DT is an improvement over the current operational strategy.

As an example, the DT, equipped with the predicted evolution of the substrate inflow, predicts a productivity/yield X of a substance obtainable at time T2. A yield Y of the substance at time T2 may be known to the operator and/or is further calculated by the DT, using the input evolution of the substrate inflow. The DT may report that Y is lower than X, signalling to the operator, a possibility for improvement by adjusting the input operational setting relating to the process parameter well ahead of reaching T2, the final/end time.

Although a comparison between values attainable using inflow of substrate is described, the embodiment is not limited to the process parameter. Additional or alternate to the substrate inflow, the DT may be input evolution of temperature between time T1 and T2, and a difference in process performance (based on any Key Performance Indicator, KPI) may be calculated by the DT based on predicted evolution of temperature and its input evolution in the bioreactor.

According to another embodiment of the invention, the determination of improvement may be carried out by comparing the predicted evolution (process performance) and the input evolution (process performance) to an optimum evolution (KPI), and determining which one of the predicted and the input evolutions converges to the optimum value.

Step 907 comprises, in case of determining an improved operation strategy by the DT, outputting the same, and/or thereafter, receiving the improved/new operating conditions at time Tx (or if real-time, at T1). Thus, the DT may be provided improved operational strategy by a human input operation, or as automatic feedback by the DT.

Thus, the control actions in the present embodiment may be effectively carried out, well ahead of the completion of the fermentation process, and the optimal operation strategy can be charted out in advance by the system or the operator. New operating conditions can be suggested to operator or changed automatically by the method during the process, so that a better/optimal process outcome may be achieved.

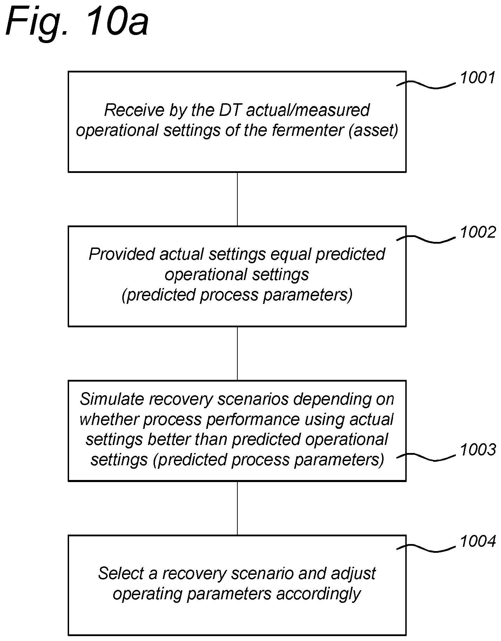

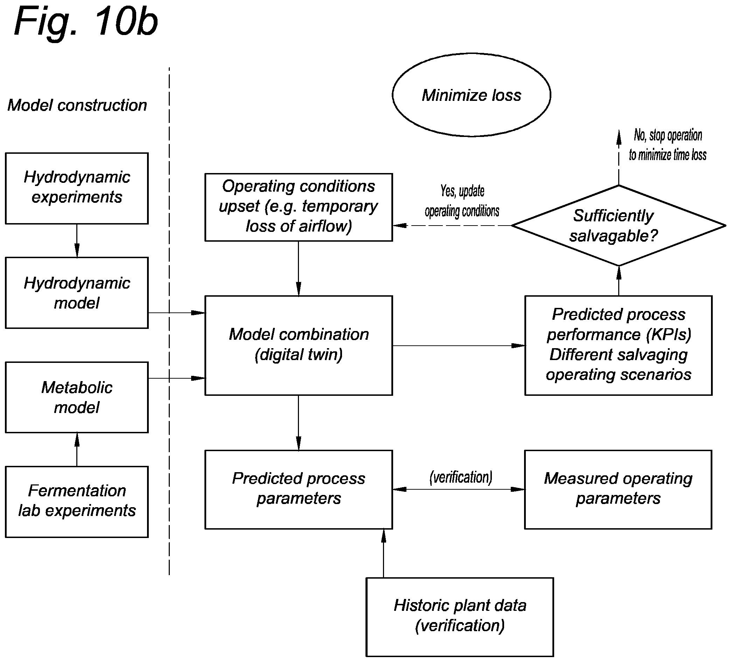

Figures 10a and 10b illustrate the use of the DT detecting and controlling process upsets/malfunctions.

Step 1001 comprises providing the DT with, or the DT receiving, actual/measured operational settings of the fermenter (asset). The operational settings may be those measured by the sensors following an upset in the bioprocess. The upset may be caused by issues like loss of airflow, pressure, stirring, feed issues, and the like. In step 901, for example, the operational settings may correspond to those existing in the bioreactor following a temporary loss of airflow.

Step 1002 comprises comparing by the DT, the provided actual settings with predicted operational settings (predicted process parameters) at time T1.

Upon determination of deviation between the measured and the predicted settings, the DT evaluates the impact of this deviation on the future bioprocess.

In step 1003, the DT predicts how the process performance evolves over time, e.g. between T1 and T2 using the input (measured) process parameters/operational settings and the operational settings predicted by the DT (the planned settings). The planned operational settings may be so designed as to incorporate an unexpected process deviation in the prediction.

Based on the above comparison, the DT further simulates multiple recovery operating scenarios, aimed at reducing the impact of the deviation on the process performance. The recovery scenarios may be determined by an algorithm, user or historical data, and are specifically designed to minimize the impact of the deviation on the product formed in the bioprocess or the process performance itself.

Each recovery scenario may correspond to a certain value of a KPI, and represents an adjustment in the operational settings to achieve the value. In step 1004, depending on which recovery scenario(s) indicates the best value to achieve an efficient process performance, the operating settings can be adjusted/updated to those corresponding to the chosen scenario.

If it is determined that none of the recovery scenarios can be used to salvage the process, the DT determines to terminate the process.

Thus, it is made feasible to determine if and when a fermentation process is not efficient, and to take control actions based on the determination. Different recovery operating conditions can be simulated, and analysed. In case of non-salvageable process upsets, the DT predicts termination of the process, which helps minimize production loss.

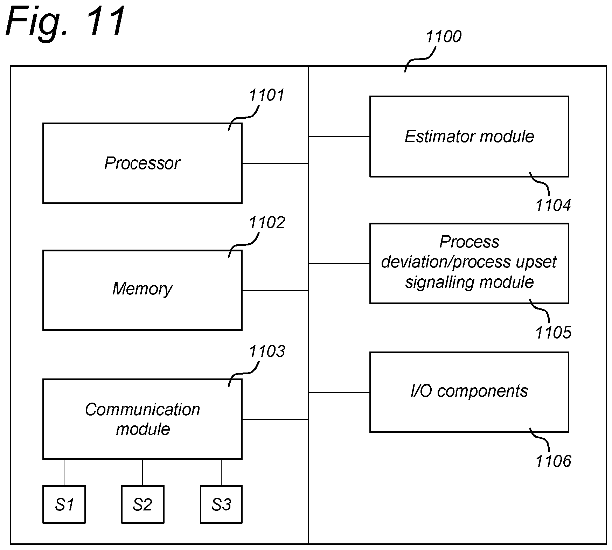

Figure 11 shows a controller device (1100) which incorporates the DT of the present invention. The device may be implemented as a standalone device, a cloud server, or a client device inter-networked with other client devices and/or a server.

The controller device (1100) may include one or more processors (1101) and associated memory devices (1102) configured to implement a plurality of computer-implemented functions, e.g. those relating to performing the steps of the present method, as illustrated in the above embodiments. The processor may include integrated circuits (ICs), microcontrollers, a programmable logic controller, application-specific processors, digital signal processors, and/or any other programmable circuits. The memory may include a volatile and/or non-volatile memory. The memory devices may include a random access memory (RAM), read only memory (ROM), one or more hard disk drives, optical drives, solid-state storage devices, and/or other suitable memory elements.

The controller may further include a communications module (1103) to facilitate communications between the controller and the various components of the bioreactor. For example, the communications module may include a sensor interface which incorporates a plurality of A/D converters to convert the signals transmitted by the one or sensors S1-S3 of the bioreactor, so that they can be processed by the controller.

The controller device may include devices such as tablet personal computers (PCs), smart phones, digital cameras, portable multimedia players (PMPs), media players, portable game consoles, and personal digital assistants (PDAs) in addition to mobile communication terminals that operate based on communication protocols corresponding to various communication systems.

The controller device may further include a number of input/output (I/O) components (1106), to receive input, provide output, transmit information, exchange information, and the like. Different kinds of input may be provided, depending on the type of device which incorporates the DT. For example, if the device is a mobile device like a hand held terminal, the device will include a touch/gesture sensitive input interface. The I/O components may further comprise visual components (e.g., a display such as a plasma display panel (PDF), a light emitting diode (LED) display, a liquid crystal display (LCD), a projector, or a cathode ray tube (CRT)), acoustic components (e.g., speakers), haptic components (e.g., a vibratory motor, resistance mechanisms), printers, other signal generators, and so forth. In additional embodiments, the I/O components may include input components like a keyboard, point based input components (e.g., a mouse, a touchpad, a trackball, or a joystick), audio input components (e.g., a microphone), and the like. The output components 1106 of the DT may be configured to display a DT user-interface, which displays process information relating to the bioprocess as well as a digital representation of the real fermenter.

The controller may further comprise an estimator module (1104) which includes the combination of the hydrodynamic and the metabolic models, and which can be configured to predict the performance of the bioprocess and the process parameters, e.g. state of the microorganism and the state of the bioreactor. The estimator module can be configured to further predict a future state of the microorganism (microbe) and the bioreactor based on operating parameters fed via the sensor interface. The estimator module may further be connected to the I/O interface of the controller for display of the output, or to transmit the output to another entity within or outside the controller for further signal processing actions.

The controller may further comprise a process deviation/process upset signalling module (1105) which can be configured to compare the output of the estimator module with that input via the sensor interface. The process deviation signalling module may further be connected to the I/O interface of the controller for display of the output, or to transmit the output to another entity within or outside the controller for further signal processing actions. The further signal processing actions may include a control action like an automatic or manual adjustment of operational parameters, the display of a notification message, e.g. flashing or sounding of a warning message for the operator, etc. The particular control action which may be implemented to signal process parameter deviation is not limited to any means.

The controller may further comprise a plurality of DTs, wherein a first DT of a first bioreactor may be communicatively coupled to a second DT of a second bioreactor in an integrated industrial facility.

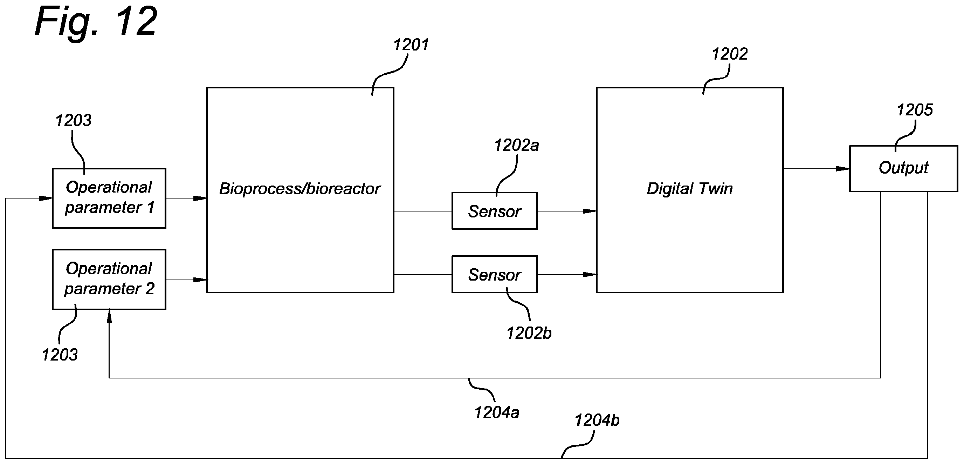

Figure 12 shows a schematic setup of such a DT used to monitor and control the industrial bioprocess.

1201 represents an industrial bioreactor whose bio(fermentation)process is controlled by the DT 1202 of the present invention.

As mentioned above, the generation and operation of the DT 1202 may be implemented by a computer system including a processor and a memory which runs software stored in it, to execute an algorithm to generate the DT and to monitor or/and control the execution of the bioprocess using the generated DT.

The fermenter 1201 further includes one or more sensors 1202a, 1202b which measure the operational settings 1203 (e.g. temperature, pressure, pH etc. inside the reactor induced at least as result of input operational parameters 1, 2) of the industrial facility during a fermentation process in the ferementer. The input operational settings 1203 may comprise at least one of operational parameters 1 and operational parameter 2, and possibly more, and may be at least one of inflow of substrate, inflow of air, air composition, outflow of gas, operating temperature or cooling flow, stirring speed, inflow of acid/base, operating pressure, etc.

Sensors 1202a, 1202b collect data and relay the operational data to the DT. Thus, according to an embodiment of an invention, there exists a fermenter in real space different from the asset used to generate the models, and providing data to a DT in a virtual space. The output of the DT may be used to monitor the real process, i.e., output data 1205 from the DT may be compared to the real process in the fermenter in real space. If unexpected deviations occur between the DT output and real process parameters, an operator may be informed by suitable reports / alarms. Alternatively or additionally, such DT output 1205 may further be used to optimise the real space in a feedback loop 1204a, 1204b. Thus, the DT in the virtual space may also provide data to the real space to improve the operational settings in real space.

The DT may be generated/implemented within near proximity to the fermenter, or in a remote device or a cloud server. In the latter case, a communication unit 12 of the asset (fermenter) receives the sensor data e.g. via a digital network, and reports the data to the DT system stored on and executed by the device/server that is communicatively coupled to the network. In an alternate embodiment, the communication unit may periodically transmit data to the cloud server via Wi-Fi, 4G or 5G, or some other form of wired or wireless communication.

The wireless communication may include cellular communication which uses at least one of, e.g., long term evolution (LTE), long term evolution- advanced (LTEA), code division multiple access (CDMA), wideband code division multiple access (WCDMA), universal mobile telecommunication system (UMTS), wireless broadband (WiBro), or global system for mobile communication (GSM). According to an embodiment of the present invention, the wireless communication may include at least one of, e.g., wireless fidelity (Wi-Fi), Bluetooth, Bluetooth low power (BLE), zigbee, near field communication (NFC), magnetic secure transmission (MST), or radio frequency network. According to an embodiment of the present invention, the wireless communication may include global navigation satellite system (GNSS). The GNSS may be, e.g., global positioning system (GPS), global navigation satellite system (Glonass), Galileo, or the European global satellite-based navigation system. The wired connection may include at least one of, e.g., universal serial bus (USB), high definition multimedia interface (HDMI), recommended standard (RS)-232, power line communication (PLC), or plain old telephone service (POTS). The network may include at least one of telecommunication networks, e.g., a computer network (e.g., local area network (LAN) or wide area network (WAN)), Internet, or a telephone network.

While the present disclosure has been described with the above described exemplary embodiments, various changes and modifications may be suggested to one skilled in the art. For example, the skilled person understands that although the invention has been described in context of a bioreactor, the method can also be used for use with any catalytic reactor. It is intended that the present disclosure encompass such changes and modifications as falling in the scope of the appended claims.

## Claims

1. A computer-implemented method for monitoring and/or controlling performance of an industrial bioprocess comprising the steps of:
providing a first model incorporating spatial variation of a process parameter in a bioreactor during the bioprocess;
providing a second model which incorporates the local response of a microorganism to the process parameter observed by said microorganism;
generating a digital twin, DT, of the bioreactor by combining the first model and the second model, wherein an output of the first model is an input to the second model and/or wherein an output of the second model is an input for the first model;
providing the DT with an input operational setting;
predicting, by the DT, a characteristic of the bioprocess based on the provided input operational setting;
monitoring and/or controlling the bioprocess based on the predicted characteristic of the bioprocess.

2. The method of claim 1, wherein the predicted characteristic of the bioprocess comprises a predicted process parameter and/or a predicted process performance.

3. Method of claim 1 or claim 2, wherein the step of predicting, by the DT, a characteristic of the bioprocess comprises predicting the characteristic of the bioprocess for more than one input operational setting.

4. The method of any of claims 1 - 3, wherein the bioprocess is controlled by tuning the input operational setting based on the predicted characteristic of the bioprocess.

5. The method of any of the preceding claims, wherein the first model comprises a hydrodynamic model which calculates a spatial variation in at least one process parameter, preferably the concentration of a substrate like sugar or dissolved oxygen, the shear rate, pH or the temperature at a predetermined location inside the bioreactor.

6. The method of any of the preceding claims, wherein the second model comprises a metabolic model which describes a state of the microorganism in the bioreactor.

7. The method of claim 1, wherein the first model and second model are combined directly, or wherein the first and second model are combined in a segregated manner.

8. The method of any of the preceding claims, wherein the method comprises measuring the input operational setting and/or process parameter via at least one measurement sensor, preferably wherein the measurement sensors comprise at least one of temperature, pressure, gas, mass analyser, velocity and pH sensors.

9. The method of any of the preceding claims, further comprising feeding the DT with the predicted characteristic of the bioprocess.

10. The method of claim 1, further comprising:
determining a deviation between the predicted characteristic and a measured characteristic of the bioprocess;
if the deviation is greater than a threshold, determining an offset in the bioprocess;
signalling the offset in the bioprocess.

11. The method of claim 1, further comprising:
providing the DT with a current time evolution and alternative time evolution of the input operational setting;
predicting a time evolution of the predicted characteristic of the bioprocess based on provided time evolutions,
determining whether the time evolution of the predicted characteristic of the bioprocess is better for an alternative than the current time evolution;
tuning the operational setting based on the determination.

12. The method of claim 1, further comprising:
detecting a process upset based on a deviation between a measured process parameter and/or operational setting and the predicted characteristic of the bioprocess;
calculating an impact of the process upset on the predicted characteristic of the bioprocess;
determining to control the bioprocess based on the calculated impact of the process upset, preferably to continue or discontinue the bioprocess.

13. The method of claim 2, wherein the predicted process parameter comprises at least one of substrate concentration, pH, temperature, dissolved oxygen, dissolved CO₂, microorganism concentration, (by)product concentration, shear stress, gas bubble size, viscosity, gas liquid mass transfer coefficient, rheology, morphology of microorganism, intracellular concentration of pools (enzymes & metabolites), local heat transfer, mass transfer, volume of broth and total volume in the bioreactor during the bioprocess.

14. The method of claim 2, wherein the predicted process performance comprises at least one of energy usage of the bioprocess, ethanol production, and yield of a product of interest.

15. The method of claim 1, wherein the input operational setting comprises at least one of inflow of substrate, inflow of air, air composition, outflow of gas, operating temperature or cooling flow, stirring speed, inflow of acid/base, and operating pressure.

16. A digital twin of bioreactor for online monitoring and/or control of an industrial bioprocess comprising:
a first compartmental model incorporating spatial variation of a process parameter in a bioreactor during the bioprocess; and
a second model which incorporates the local response of a microorganism to the process parameter observed by said microorganism, wherein the digital twin is configured to performed method steps corresponding to any of claims 1-15.

17. A computer program product comprising instructions which, when the program is executed by a computer, causes the computer to carry out the method steps of any one of claims 1-15.

## Patentansprüche

1. Computerimplementiertes Verfahren zur Überwachung und/oder Steuerung der Leistung eines industriellen Bioprozesses, umfassend die folgenden Schritte:
Bereitstellen eines ersten Modells, das räumliche Variation eines Prozessparameters in einem Bioreaktor während des Bioprozesses berücksichtigt;
Bereitstellen eines zweiten Modells, das die lokale Reaktion eines Mikroorganismus auf den Prozessparameter berücksichtigt, der durch den Mikroorganismus beobachtet wird;
Erzeugen eines digitalen Zwillings, DT, des Bioreaktors durch Kombinieren des ersten Modells und des zweiten Modells, wobei eine Ausgabe des ersten Modells eine Eingabe in das zweite Modell ist, und/oder wobei eine Ausgabe des zweiten Modells eine Eingabe für das erste Modell ist;
Bereitstellen einer Eingangsbetriebseinstellung für den DT;
Vorhersagen einer Charakteristik des Bioprozesses durch den DT basierend auf der bereitgestellten Eingangsbetriebseinstellung;
Überwachen und/oder Steuern des Bioprozesses basierend auf der vorhergesagten Charakteristik des Bioprozesses.

2. Verfahren nach Anspruch 1, wobei die vorhergesagte Charakteristik des Bioprozesses einen vorhergesagten Prozessparameter und/oder eine vorhergesagte Prozessleistung umfasst.

3. Verfahren nach Anspruch 1 oder 2, wobei der Schritt des Vorhersagens einer Charakteristik des Bioprozesses durch den DT ein Vorhersagen der Charakteristik des Bioprozesses für mehr als eine Eingangsbetriebseinstellung umfasst.

4. Verfahren nach einem der Ansprüche 1 bis 3, wobei der Bioprozess durch Abstimmen der Eingangsbetriebseinstellung basierend auf der vorhergesagten Charakteristik des Bioprozesses gesteuert wird.

5. Verfahren nach einem der vorhergehenden Ansprüche, wobei das erste Modell ein hydrodynamisches Modell umfasst, das eine räumliche Variation mindestens eines Prozessparameters, vorzugsweise der Konzentration eines Substrats wie Zucker oder gelösten Sauerstoffs, der Scherrate, des pH oder der Temperatur an einer vorbestimmten Stelle innerhalb des Bioreaktors, berechnet.

6. Verfahren nach einem der vorhergehenden Ansprüche, wobei das zweite Modell ein Stoffwechselmodell umfasst, das einen Zustand des Mikroorganismus im Bioreaktor beschreibt.

7. Verfahren nach Anspruch 1, wobei das erste Modell und das zweite Modell direkt kombiniert werden, oder wobei das erste und das zweite Model in getrennter Weise kombiniert werden.

8. Verfahren nach einem der vorhergehenden Ansprüche, wobei das Verfahren ein Messen der Eingangsbetriebseinstellung und/oder des Prozessparameters mittels mindestens eines Messsensors umfasst, wobei die Messsensoren vorzugsweise mindestens einen von Temperatur-, Druck-, Gas-, Massenanalysator-, Geschwindigkeits- und pH-Sensoren umfassen.

9. Verfahren nach einem der vorhergehenden Ansprüche, ferner umfassend ein Eingeben der vorhergesagten Charakteristik des Bioprozesses in den DT.

10. Verfahren nach Anspruch 1, ferner umfassend:
Bestimmen einer Abweichung zwischen der vorhergesagten Charakteristik und einer gemessenen Charakteristik des Bioprozesses;
Bestimmen eines Versatzes im Bioprozess, falls die Abweichung über einer Schwelle liegt;
Signalisieren des Versatzes im Bioprozess.

11. Verfahren nach Anspruch 1, ferner umfassend:
Bereitstellen einer aktuellen Zeitentwicklung und einer alternativen Zeitentwicklung der Eingangsbetriebseinstellung für den DT;
Vorhersagen einer Zeitentwicklung der vorhergesagten Charakteristik des Bioprozesses basierend auf den bereitgestellten Zeitentwicklungen,
Bestimmen, ob die Zeitentwicklung der vorhergesagten Charakteristik des Bioprozesses besser ist für eine Alternative als die aktuelle Zeitentwicklung;
Abstimmen der Betriebseinstellung basierend auf der Bestimmung.

12. Verfahren nach Anspruch 1, ferner umfassend:
Erkennen einer Prozessstörung basierend auf einer Abweichung zwischen einem gemessenen Prozessparameter und/oder einer Betriebseinstellung und der vorhergesagten Charakteristik des Bioprozesses;
Berechnen einer Auswirkung der Prozessstörung auf die vorhergesagte Charakteristik des Bioprozesses;
Bestimmen, den Bioprozess basierend auf der berechneten Auswirkung der Prozessstörung zu steuern, vorzugsweise den Prozess fortzusetzen oder zu unterbrechen.

13. Verfahren nach Anspruch 2, wobei der vorhergesagte Prozessparameter mindestens eines von Substratkonzentration, pH, Temperatur, gelöstem Sauerstoff, gelöstem CO₂, Konzentration von Mikroorganismen, (Neben-)Produktkonzentration, Scherrate, Gasblasengröße, Viskosität, Gas-Flüssigkeit-Massentransferkoeffizient, Rheologie, Morphologie von Mikroorganismen, intrazellulärer Konzentration von Pools (Enzymen und Metaboliten), lokale Wärmeübertragung, Massentransfer, Volumen von Brühe und Gesamtvolumen im Bioreaktor während des Bioprozessors umfasst.

14. Verfahren nach Anspruch 2, wobei die vorhergesagte Prozessleistung mindestens eines von Energienutzung des Bioprozesses, Ethanolproduktion und Ausbeute eines Produkts von Interesse umfasst.

15. Verfahren nach Anspruch 1, wobei die Eingangsbetriebseinstellung mindestens eines von Substratzufluss, Luftzufluss, Luftzusammensetzung, Gasausfluss, Betriebstemperatur oder Kühlfluss, Rührgeschwindigkeit, Säure-/Basenzufluss und Betriebsdruck umfasst.

16. Digitaler Zwilling eines Bioreaktor zur Online-Überwachung und/oder -Steuerung eines industriellen Bioprozesses, umfassend:
ein erstes Kompartimentmodell, das räumliche Variation eines Prozessparameters in einem Bioreaktor während des Bioprozesses berücksichtigt; und
ein zweites Kompartimentmodell, das die lokale Reaktion eines Mikroorganismus auf den Prozessparameter berücksichtigt, der durch den Mikroorganismus beobachtet wird, wobei der digitale Zwilling zum Ausführen der Verfahrensschritte nach einem der Ansprüche 1 bis 15 konfiguriert ist.

17. Computerprogrammprodukt, umfassend Anweisungen, die bei Ausführung des Programms durch einen Computer den Computer zum Ausführen der Verfahrensschritte nach einem der Ansprüche 1 bis 15 veranlassen.

## Revendications

1. Procédé mis en œuvre par ordinateur pour surveiller et/ou commander les performances d'un bioprocédé industriel, comprenant les étapes de :
fourniture d'un premier modèle incorporant la variation spatiale d'un paramètre de processus dans un bioréacteur pendant le bioprocédé ;
fourniture d'un second modèle qui incorpore la réponse locale d'un micro-organisme au paramètre de processus observé par ledit micro-organisme ;
génération d'un double numérique, DT, du bioréacteur en combinant le premier modèle et le second modèle, une sortie du premier modèle étant une entrée pour le second modèle et/ou une sortie du second modèle étant une entrée pour le premier modèle ;
fourniture au DT d'un réglage opérationnel d'entrée ;
prédiction, par le DT, d'une caractéristique du bioprocédé sur la base du paramètre opérationnel d'entrée fourni ;
surveillance et/ou commande du bioprocédé sur la base de la caractéristique prédite du bioprocédé.

2. Procédé selon la revendication 1, la caractéristique prédite du bioprocédé comprenant un paramètre de processus prédit et/ou une performance de processus prédite.

3. Procédé selon la revendication 1 ou selon la revendication 2, l'étape de prédiction, par le DT, d'une caractéristique du bioprocédé comprenant la prédiction de la caractéristique du bioprocédé pour plus d'un paramètre opérationnel d'entrée.

4. Procédé selon l'une quelconque des revendications 1 à 3, le bioprocédé étant commandé en accordant le réglage opérationnel d'entrée sur la base de la caractéristique prédite du bioprocédé.

5. Procédé selon l'une quelconque des revendications précédentes, le premier modèle comprenant un modèle hydrodynamique qui calcule une variation spatiale d'au moins un paramètre du processus, de préférence la concentration d'un substrat comme le sucre ou l'oxygène dissous, le taux de cisaillement, le pH ou la température à un emplacement prédéterminé à l'intérieur du bioréacteur.

6. Procédé selon l'une quelconque des revendications précédentes, le second modèle comprenant un modèle métabolique qui décrit un état du micro-organisme dans le bioréacteur.

7. Procédé selon la revendication 1, le premier modèle et le second modèle étant combinés directement, ou le premier et le second modèle étant combinés de manière séparée.

8. Procédé selon l'une quelconque des revendications précédentes, le procédé comprenant la mesure du paramètre opérationnel d'entrée et/ou du paramètre de processus par l'intermédiaire d'au moins un capteur de mesure, de préférence les capteurs de mesure comprenant au moins l'un des capteurs de température, de pression, de gaz, d'analyseur de masse, de vitesse et de pH.

9. Procédé selon l'une quelconque des revendications précédentes, comprenant en outre l'alimentation du DT avec la caractéristique prédite du bioprocédé.

10. Procédé selon la revendication 1, comprenant en outre :
la détermination d'un écart entre la caractéristique prédite et une caractéristique mesurée du bioprocédé ;
si l'écart est supérieur à un seuil, la détermination d'un décalage dans le bioprocédé ;
la signalisation du décalage dans le bioprocédé.

11. Procédé selon la revendication 1, comprenant en outre :
la fourniture au DT d'une évolution temporelle actuelle et d'une évolution temporelle alternative du paramètre opérationnel d'entrée ;
la prédiction d'une évolution temporelle de la caractéristique prédite du bioprocédé sur la base des évolutions temporelles fournies,
la détermination si l'évolution temporelle de la caractéristique prédite du bioprocédé est meilleure pour une alternative que l'évolution temporelle actuelle ;
le fait d'accorder le paramètre opérationnel sur la base de la détermination.

12. Procédé selon la revendication 1, comprenant en outre :
la détection d'une perturbation du processus sur la base d'un écart entre un paramètre de processus mesuré et/ou un réglage opérationnel et la caractéristique prédite du bioprocédé ;
le calcul d'un impact de la perturbation du processus sur la caractéristique prédite du bioprocédé ;
la détermination de la commande du bioprocédé sur la base de l'impact calculé de la perturbation du processus, de préférence pour continuer ou interrompre le bioprocédé.

13. Procédé selon la revendication 2, le paramètre de processus prédit comprenant au moins l'un de la concentration de substrat, du pH, de la température, de l'oxygène dissous, du CO₂ dissous, de la concentration de micro-organisme, de la concentration de (sous)produit, de la contrainte de cisaillement, de la taille de bulle de gaz, de la viscosité, du coefficient de transfert de masse gaz-liquide, de la rhéologie, de la morphologie du micro-organisme, de la concentration intracellulaire de groupes (enzymes et métabolites), du transfert de chaleur local, du transfert de masse, du volume de bouillon de culture et du volume total dans le bioréacteur pendant le bioprocédé.

14. Procédé selon la revendication 2, la performance prédite du processus comprenant au moins l'un de la consommation d'énergie du bioprocédé, de la production d'éthanol et du rendement d'un produit d'intérêt.

15. Procédé selon la revendication 1, le paramètre opérationnel d'entrée comprenant au moins un élément parmi l'entrée de substrat, l'entrée d'air, la composition de l'air, la sortie de gaz, la température de fonctionnement ou le flux de refroidissement, la vitesse d'agitation, l'entrée d'acide/base et la pression de fonctionnement.

16. Double numérique de bioréacteur pour la surveillance et/ou la commande en ligne d'un bioprocédé industriel comprenant :
un premier modèle compartimental incorporant la variation spatiale d'un paramètre de processus dans un bioréacteur au cours du bioprocédé ; et
un second modèle qui incorpore la réponse locale d'un microorganisme au paramètre de processus observé par ledit microorganisme, le double numérique étant configuré pour réaliser les étapes du procédé correspondant à l'une quelconque des revendications 1 à 15.

17. Produit de programme informatique comprenant des instructions qui, lorsque le programme est exécuté par un ordinateur, amènent l'ordinateur à réaliser les étapes du procédé selon l'une quelconque des revendications 1 à 15.
